# EUROPEAN PATENT SPECIFICATION

(11) **EP 4 447 878 B1**
(45) Date of publication and mention of the grant of the patent: **12.11.2025**
(21) Application number: 22851412.1
(22) Date of filing: 15.12.2022
(51) Int. Cl.: A61F 2/95

(54) **STENT GRAFT CRIMPING DEVICE AND METHOD OF USE**
VORRICHTUNG ZUM KOMPRIMIEREN EINES STENTIMPLANTATS UND VERFAHREN ZUR VERWENDUNG
DISPOSITIF DE SERTISSAGE DE GREFFE D'ENDOPROTHÈSE ET PROCÉDÉ D'UTILISATION

(30) Priority: 16.12.2021 US 202163290344 P
(43) Date of publication of application: 23.10.2024
(73) Proprietor: Bolton Medical, Inc., Sunrise, FL 33325 (US)
(72) Inventor: VANCHERI, Bryan, Sunrise, FL 33325 (US)
(74) Representative: Graham Watt & Co
(86) International application number: PCT/US2022/052968
(87) International publication number: WO 2023/114371

(56) References cited:
- US-A- 5 836 952
- US-A- 6 141 855
- US-A1- 2004 148 007
- US-A1- 2011 295 361

## Description

### CROSS-REFERENCE TO RELATED APPLICATION

This application claims the benefit of U.S. Provisional Patent Application No. 63/290,344, filed December 2021.

### BACKGROUND OF THE INVENTION

Demonstration of stent graft delivery devices typically entails release of radially constricted, or "crimped," stent grafts that have previously been loaded within one or more delivery or "introducer" sheaths. Most stent grafts delivered by such devices are self-expanding and include a plurality of radially self-expanding stents, often fabricated of intermetallic or shape memory alloys. Once deployed in the course of demonstrating a surgical delivery device within which they had been packaged during assembly and fabrication, self-expanding stent graft typically are very difficult to reload by hand.

Known devices for loading surgical stent graft prostheses into surgical delivery devices, such as "iris crimpers," are complex, heavy, expensive, and too large for transport by personnel responsible for sales and marketing at locations remote from the place of their manufacture.

US2011/295361 A1 discloses a catheter deliverable artificial multi-leaflet heart valve prosthesis and intravascular delivery system for a catheter deliverable heart valve prosthesis.

US 5 836 952 discloses a hand-held stent crimper.

Therefore, a need exists for a device, system, and method of loading stent grafts into surgical delivery devices in the field.

### SUMMARY OF THE INVENTION

The invention generally is directed to a stent graft crimping device and system for radially constricting a stent graft, and a method of loading a stent graft into a stent graft delivery device.
In one embodiment, the invention is a stent graft crimping device for radially constricting the stent graft that includes a rigid cylinder and a flexible sheet. The rigid cylinder has an outside surface and an inside surface, and defines a lumen and a slot extending parallel to a longitudinal axis of the rigid cylinder. The flexible sheet has a straight edge, a raised component at the straight edge, and a second edge opposite the straight edge. The raised component and the straight edge are parallel to and adjacent to the longitudinal slot at the outside surface of the rigid cylinder. The flexible sheet extends from the straight edge and through the slot into the lumen of the rigid cylinder, and from the lumen through the longitudinal slot to the second edge of the outside surface of the rigid cylinder, thereby defining a pocket of the flexible sheet within the lumen of the rigid cylinder.

In another embodiment, the invention is a stent graft loading system that includes a rigid cylinder, a flexible sheet, a guidewire catheter, an apex clasping component, and an apex clasping catheter. The rigid cylinder has an outside surface and an inside surface, and defines a lumen and a slot extending parallel to a longitudinal axis of the rigid cylinder. The flexible sheet has a straight edge, a raised component at the straight edge, and a second edge opposite the straight edge. The raised component and the straight edge are parallel to and adjacent to the longitudinal slot at the outside surface of the rigid cylinder. The flexible sheet extends from the straight edge and through the slot into the lumen of the rigid cylinder, and from the lumen through the longitudinal slot to the second edge at the outside surface of the rigid cylinder, thereby defining a pocket of the flexible sheet within the lumen of the rigid cylinder. The guidewire catheter extends through the pocket and has a distal end and a proximal end. The apex clasping component is fixed to the distal end of the guidewire catheter, and includes a distal clasping component at the distal end of the guidewire catheter and a proximal clasping component that is mateable to the distal clasping component and proximal to the distal clasping component. The apex clasping catheter is fixed to and extends proximally from the proximal clasping component of the apex clasping component.

In still another embodiment, the invention is a method of loading a stent graft into a stent graft delivery device and includes the step of loading the stent graft into a pocket defined by a flexible sheet having a straight edge and a second edge opposite the straight edge, wherein the straight edge has a raised component that extends parallel to and is adjacent a slot defined by a rigid cylinder, and extends parallel to a longitudinal axis of the rigid cylinder. The flexible sheet extends from the raised component of the straight edge through the slot and into a lumen defined by the rigid cylinder, and from within the lumen through the slot to the opposite edge of the flexible sheet outside the rigid cylinder to thereby define the pocket of the flexible sheet. The second edge of the flexible sheet is pulled away from the rigid cylinder, thereby reducing the volume of the pocket and radially constricting the stent graft and causing a longitudinal axis of the stent graft to move away from the longitudinal axis of the rigid cylinder and toward the slot of the rigid cylinder. The stent graft is directed along its longitudinal axis from the pocket into an independent radially constricting member while remaining radially constricted along its longitudinal axis.

This invention has many advantages. For example, the stent graft crimping device of the invention is simple to use and easily transportable. Further, the stent graft loading system and method of loading the stent graft into a stent graft delivery device of the invention can accommodate different types of stent grafts, such as those that employ longitudinal supports sewn into a fabric of the stent graft, including support bars, such as longitudinal support bars, and stent grafts that employ crown and clasping stents that must be mounted to apex clasping components of surgical stent graft delivery devices. Also, stent grafts that require external longitudinal sport, such as support wires that are fixed to and extend distally along other components of the surgical stent graft delivery device and are threaded through suture loops of the stent graft, also can be accommodated by the stent graft crimping device, system and method of use of the invention.

### BRIEF DESCRIPTION OF THE DRAWINGS

FIG. 1A is a perspective view of a rigid cylinder component of one embodiment of a stent graft crimping device of the invention.
FIG. 1B is an end view of the rigid cylinder shown in FIG. 1A.
FIG. 2A is a plan view of a flexible sheet component of one embodiment of a stent graft crimping device of the invention.
FIG. 2B is an edge view of the flexible sheet shown in FIG. 2A
FIG. 3A is a perspective view of the assembled stent graft crimping device shown in FIGs. 1A, 1B, 2A, and 2B prior to radial constriction of a pocket defined by the flexible sheet of the stent graft crimping device of the invention.
FIG. 3B is an end view of the stent graft crimping device of the invention shown in FIG. 3A.
FIG. 4A is a perspective view of the stent graft crimping device of the invention shown in FIGs. 3A and 3B following radial constriction of the pocket defined by the flexible sheet.
FIG. 4B is an end in view of the stent graft crimping device of the invention shown in FIG. 4A.
FIG. 5A is a perspective view of the stent graft crimping device shown in FIGs. 1A through 4B wherein a stent graft has been loaded into the pocket defined by the flexible sheet, but prior to radially constricting the stent graft by an embodiment of a method of the invention.
FIG. 5B is an end view of the stent graft crimping device and loaded stent graft prosthesis of FIG. 5A.
FIG. 6A is a perspective view of the stent graft crimping device shown in FIGs. 5A and 5B, but following radial constriction of the stent graft by the method of the invention.
FIG. 6B is an end view of the stent graft crimping device shown in FIG. 6A.
FIG. 7 is an exploded view, in perspective, of another embodiment of the stent graft crimping device of the invention, further including a loading block and a loading tube.
FIG. 8 is an assembled view of the stent graft crimping device of FIG.7.
FIG. 9 is a detail of another embodiment of a stent graft loading system of the invention, showing a proximal end of a stent graft partially withdrawn from a rigid cylinder in order to mount a clasping stent of the stent graft onto an apex clasping component of the stent graft loading system, and sutures of the stent graft onto support wires of the stent graft loading system.
FIG. 10 is one embodiment of an apex clasping component of the stent graft loading system of FIG. 9 in a closed position.
FIG. 11 is a perspective view of a loading rod of the stent graft loading system of FIG. 9 that is threadably fixable to a guidewire catheter of a stent graft delivery device suitable for use with the invention.
FIG. 12 is a perspective view of a stent graft suitable for use with the invention that includes a crown stent, and a clasping stent with bare proximal apices that can be captured by an apex clasping component, also shown in FIG.12.
FIG. 13 is a perspective view of the stent graft of FIG. 12 following capture by the apex clasping component.
FIG. 14 is a perspective view of a stent graft that includes suture loops that are threadable by wire supports of a delivery device suitable for use with the stent graft loading system of the invention.
FIG. 15 is a perspective view of the stent graft of FIG. 14 while the suture loops are being threaded by the support wires that are components of this embodiment of the stent graft loading system of the invention.
FIG. 16 is an end view of a stent graft of the invention that includes an S-bar support as loaded within a pocket defined by a flexible sheet of a stent graft crimping device of the invention.
FIG. 17 is a side view of one embodiment of a stent graft delivery device that is suitable for use with the stent graft crimping device and stent graft loading system of the invention.
FIG. 18 is a perspective view of one embodiment of a method step of the invention including loading a radially constricted stent graft from rigid cylinder, through a loading block, and into a loading tube of one embodiment of the stent graft crimping device of the invention.
FIG. 19 is a perspective view of one embodiment of two additional method steps following that shown in FIG.18, wherein the loading tube is substituted with a secondary sheath of a stent graft delivery device suitable for use with the stent graft crimping device of the invention.

### DETAILED DESCRIPTION OF THE INVENTION

The features and other details of the invention, either as steps of the invention or as combinations of parts of the invention will now be more particularly described and pointed out in the claims. It will be understood that the particular embodiments of the invention are shown by way of illustration and not as limitations of the invention. The principle features of this invention can be employed in various embodiments without departing from the scope of the invention.

A description of example embodiments follows.

The invention generally is directed to a stent graft crimping device and system for radially constricting a stent graft, and to a method of loading a stent graft into a stent graft delivery device.

When reference is made herein to an aortic prosthesis, such as a "stent graft," "prosthesis," "stent graft prosthesis," "vascular prosthesis" or other prostheses to be delivered or implanted in a patient, the word "proximal" means that portion of the prosthesis or component of the prosthesis that is relatively close to the heart of the patient, while "distal" means that portion of the prosthesis or component of the prosthesis that is relatively far from the heart of the patient.

When, however, reference is made to a delivery system or a component of a delivery system employed to deliver, or implant, a prosthesis, the word, "proximal," as that word is employed herein, means closer to the clinician using the delivery system. When reference is made to a delivery system where a component of the delivery system is "distal," as that term is employed herein, means further away from the clinician using the delivery system.

For clarity, the word "proximate" means "close to," as opposed to the meanings ascribed to "proximal" or "distal" described above with respect to either the prosthesis or delivery system.

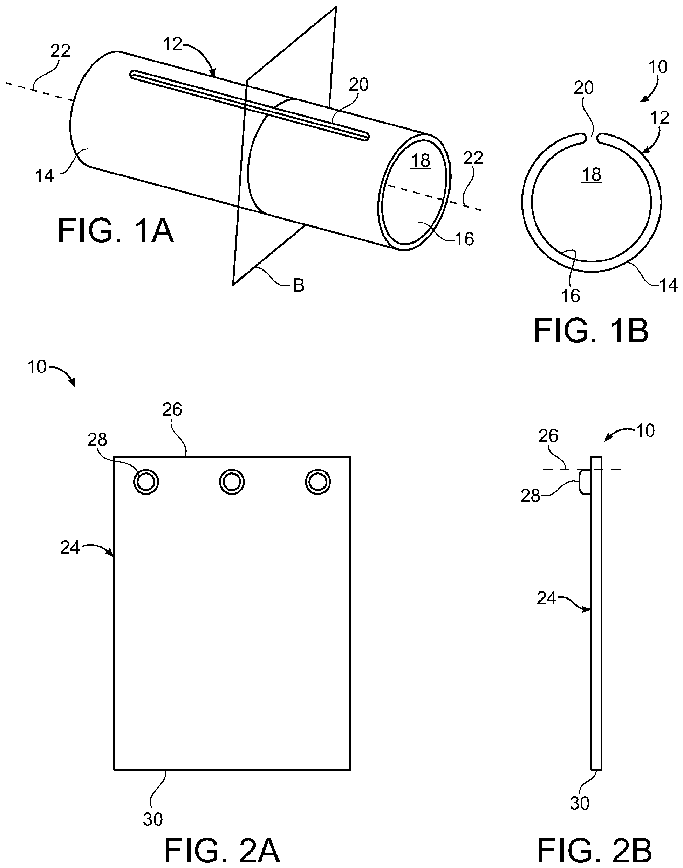

Component parts of one embodiment of a stent graft crimping device 10 of the invention for radially constricting a stent graft is shown in FIGs. 1A, 1B, 2A, 2B. As can be seen in the perspective view of FIG. 1A, and the end view of FIG. 1B taken along plane B of FIG. 1A, stent graft crimping device 10 includes rigid cylinder 12 having outside surface 14 and inside surface 16, and defines lumen 18 and slot 20 extending parallel to longitudinal axis 22 of rigid cylinder 12. An example of a suitable material of construction of rigid cylinder 12 is clear acrylic. Flexible sheet 24, shown in plan and edge views in FIGs. 2A and 2B, respectively, has straight edge 26 and raised component 28 at straight edge 26, and second edge 30 that is opposite straight edge 26. An example of a suitable material of construction of flexible sheet is Teflon PTFE film having a thickness in a range of between about 0.010 inches and about 0.050 inches, in a preferred embodiment about 0.010 inches or 0.010 inches. Raised component 28 of flexible sheet 24 protrudes from at least one side of flexible sheet 24, as can be seen in FIG. 2B. Typically, raised component 28 is a nylon two-piece push-in rivet with a snap shank.

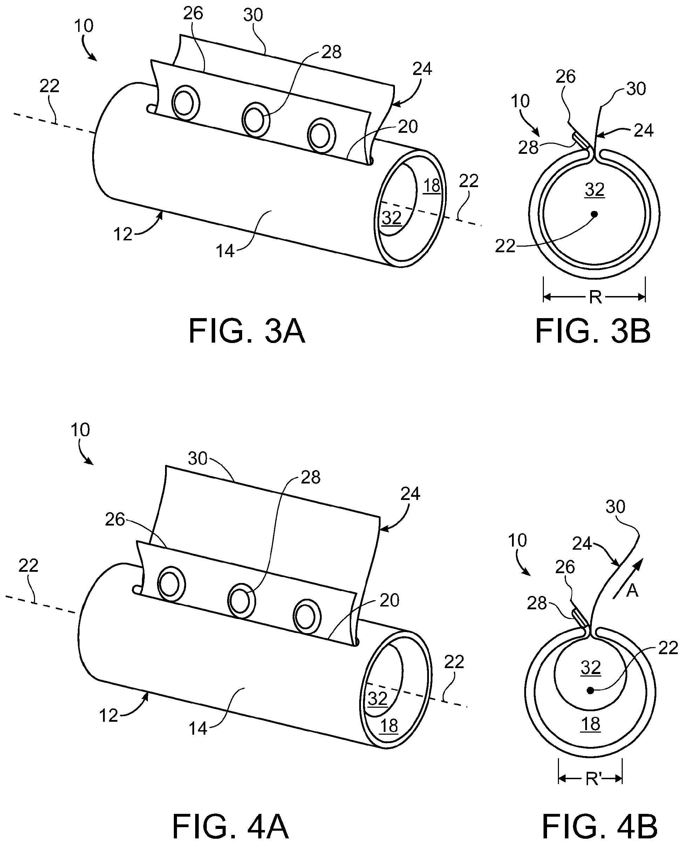

When assembled, as shown in FIGs 3A and 3B, raised component 28 of straight edge 26 and second edge 30 are parallel to and adjacent to longitudinal slot 20 at outside surface 14 of rigid cylinder 12. Flexible sheet 24 extends from straight edge 26 and through longitudinal slot 20 into lumen 18 of rigid cylinder 12, and from lumen 18 through longitudinal slot 20 to second edge 30 at outside surface 14 of rigid cylinder 12, thereby defining pocket 32 of flexible sheet 24 within lumen 18 of rigid cylinder 12.

As demonstrated in the transition from FIGs. 3A and 3B to FIGs. 4A and 4B, pocket 32 defined by flexible sheet 24 reduces in radial diameter R of pocket 32 to reduced radial diameter R' by pulling second edge 30 in direction A away from longitudinal slot 20 of rigid cylinder 12, such as by hand. Raised component 28 of flexible sheet 24 prevents straight edge 26 from being pulled into lumen 18 of rigid cylinder 12, and friction between contacting surfaces at flexible sheet 24 at longitudinal slot 20 where flexible sheet 24 passes through longitudinal slot 20 can provide some resistance to expansion of pocket 32 once it has been radially constricted by pulling of second edge 30 of flexible sheet 24.

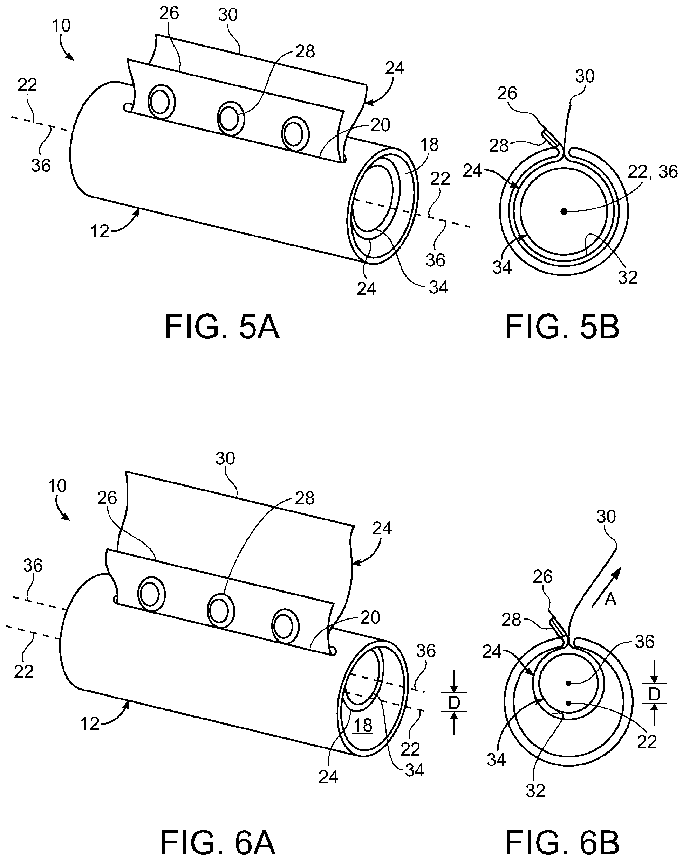

FIGs. 5A and 5B, and 6A and 6B show the embodiment of the stent graft crimping device of the invention of FIGs. 1A-4B upon loading with radially self-expandable stent graft 34. As can be seen in FIG. 5B, radially self-expanding stent graft 34 is in a relatively relaxed state within pocket 32 defined by flexible sheet 24 within lumen of rigid cylinder 12 prior to radial constriction by the method of the invention. Longitudinal axis 22 of rigid cylinder 12 and longitudinal axis 36 of radially self-expandable stent graft 34 can coincide, as shown in FIG. 5B.

Upon pulling second edge 30 of flexible sheet 24 away from longitudinal slot 20 of rigid cylinder 12, as shown in the transition from FIG. 5A to FIG. 6A, radially self-expanding prosthesis 34 becomes radially constricted, as shown in the progression from FIG. 5B to FIG. 6B. As radially self-expanding stent graft 34 becomes radially constricted, longitudinal axis 36 of radially self-expanding stent graft 34 moves away from longitudinal axis 22 of rigid cylinder 12. In one instance, shown in FIGs. 5A and 5B, where longitudinal axes 22, 36 of rigid cylinder 12 and radially self-expanding stent graft 34, respectively, coincide, longitudinal axis 36 of radially self-expanding prosthesis 34 moves distance D from longitudinal axis 22 of rigid cylinder 12 during radial constriction caused by pulling second edge 30 away from longitudinal slot 20 of rigid cylinder 12.

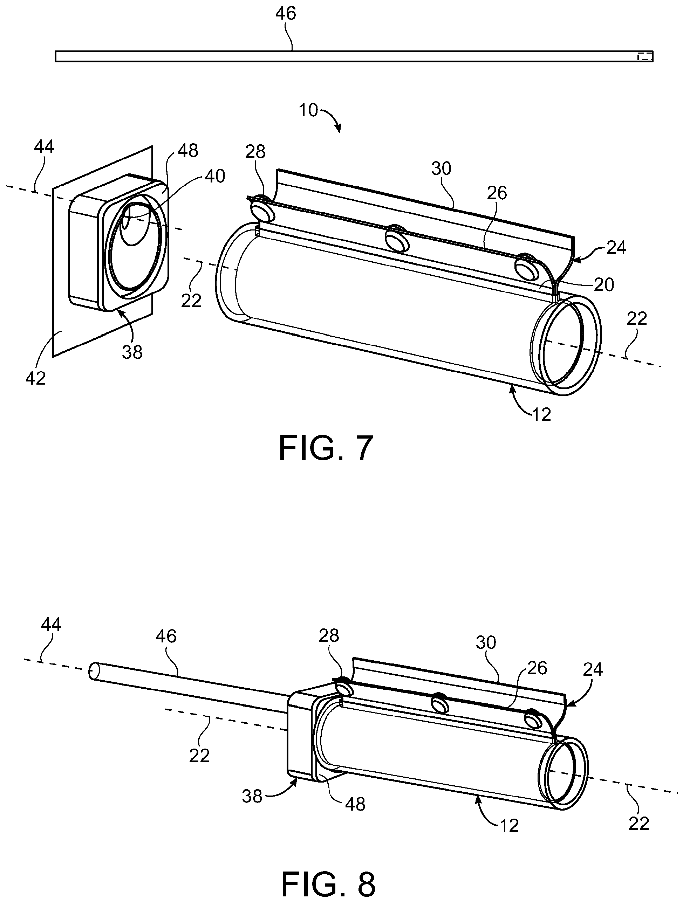

FIG. 7 is an exploded view of one specific embodiment of a stent graft crimping device of the invention, further including loading block 38 mateable to rigid cylinder 12. Loading block 38 typically is formed by 3D printing using an acrylonitrile butadiene styrene (ABS)-type material. Loading block 38 defines opening 40 in a plane 42 that is perpendicular to longitudinal axis 21 of rigid cylinder 12. Opening 40 defines center axis 44 that is parallel to longitudinal axis 22 of rigid cylinder 12, but spaced apart from longitudinal axis 22 of rigid cylinder 22. In one specific embodiment, loading tube 46 extends from opening 40 at loading block 38 opposite to portion 48 of loading block 38 that is mated with rigid cylinder 12, as shown in FIG.8, by suitable means, such as by an interference fit. Loading tube 46 typically is matched by an interference fit with loading block 38 and is formed of clear PTFE tubing. Loading tube 46 can be an intermediate means, whereby radially compacted radially self-expandable prosthesis 34, once delivered to loading tube 46, is then sheathed by substitution of loading tube 46 with a secondary sheath of surgical delivery device (not shown).

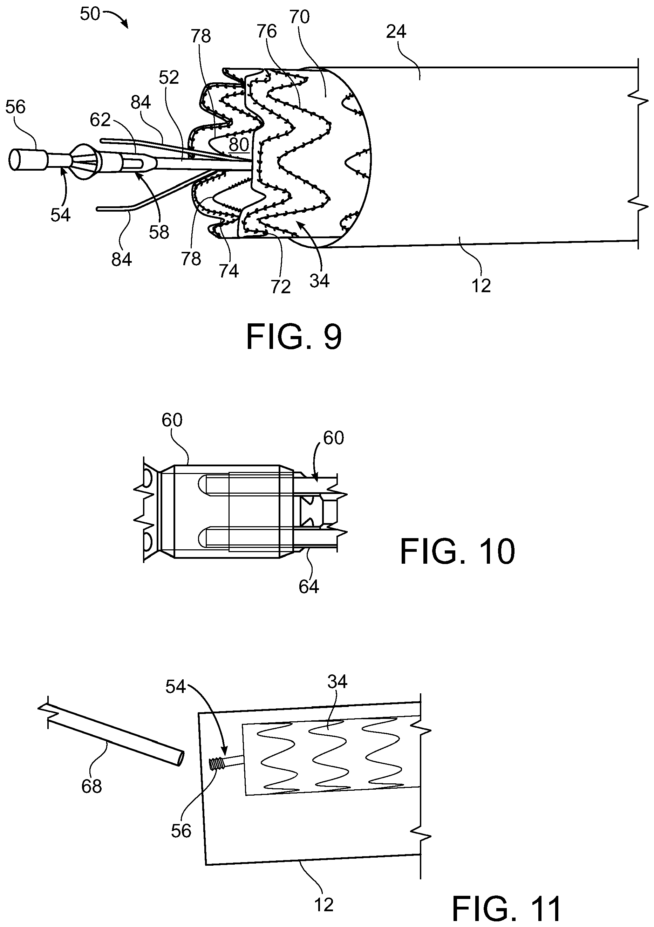

In another embodiment of the invention, stent graft loading system 50 includes rigid cylinder 12 and flexible sheet 24, and also at least one component of a stent graft delivery device. As shown in FIG.9, for example, stent graft loading system 50 includes, in addition to rigid cylinder 12 and flexible sheet 24, guidewire catheter (not shown) extending through apex clasping catheter 52. Guidewire catheter 54 has distal end 56 and a proximal end (not shown), and is part of a prosthesis delivery device employed by a surgeon or for demonstration by field personnel. Apex clasping component 58 of stent graft loading system 50 includes distal clasping component 60 that is fixed at distal end 56 of guidewire catheter 54. Proximal clasping component 62 of apex clasping component 58 includes tines 64 that extend distally from proximal clasping component 62. Apex clasping catheter 52 extends proximally from proximal clasping component 62 of apex clasping component 58. Proximal clasping component 62 is mateable to distal clasping component 60, as shown in FIG.10. Examples of suitable clasping components can be found in U.S. 8,292,943 and U.S. 10,646,365, the entire teachings of which are incorporated by reference in their entirety.

As can be seen in FIG.11, in one specific embodiment, loading rod 68 is releasably fixable to distal end 56 of guidewire catheter 54, such as by threading onto threaded distal end 56 of guidewire catheter 54 prior to fully assembling the delivery device. Loading rod 68 typically is formed of stainless steel.

In one embodiment of stent graft loading system 50 of the invention, radially self-expandable stent graft 34 is within pocket 32 defined by flexible sheet 24, but can be partially withdrawn from pocket 32, as shown, for example, in FIG.9, to allow axis to components of stent graft delivery system that are also components of the stent graft loading system 50 of the invention. Specifically, in one embodiment, radially self-expandable stent graft 34 includes luminal graft component 70 having proximal end 72 and distal end (not shown). As shown in FIG.9, radially self-expandable stent graft 34 includes crown stent 74 fixed to proximal end 72 of luminal graft component 70. Crown stent 74 is proximal and adjacent to clasping stent 76. Clasping stent 76 includes at least one bare apex 78 that is exposed to lumen 80 of luminal graft component 70.

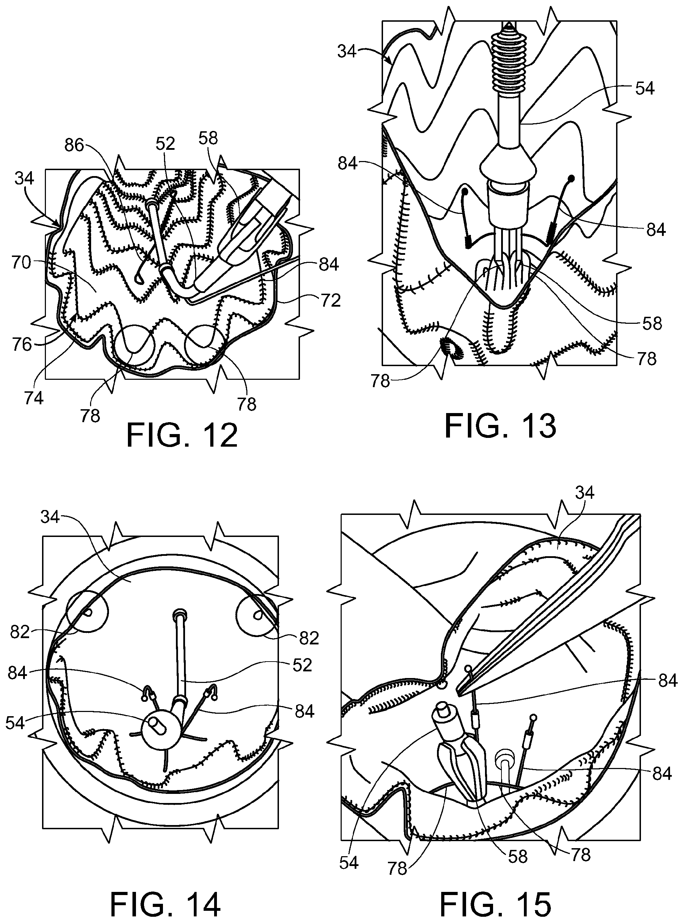

In the embodiment shown in FIGs. 9 through 13, proximal clasping component includes tines 64, or prongs, that extend distally from proximal clasping component 62 and are mateable with distal clasping component 60, whereby at least one bare proximal apex 78 of clasping stent 76 can be captured between tines 64 of proximal clasping component 62 and distal clasping component 60, as shown in FIG.13, by distal movement of apex capture catheter 52 to move tines 64 of distal clasping component 60 through the at least one bare apex 78 of clasping stent 76 and into mating relation with distal apex clasping component 60 to thereby capture bare proximal apex 78. One bare apex is captured by each prong of proximal apex clasping component 62. The at least one bare proximal apex 78 can then be released by proximal movement of apex clasping catheter 52, to thereby move proximal clasping component 62 out of mating relation with distal clasping component 60.

In another embodiment, independently or in combination with any of the embodiments described above, radially self-expandable stent graft 34 includes at least one suture loop 82 at proximal end 72 of radially self-expandable stent graft 34 and within radially self-expandable stent graft 34, as shown in FIG.14. In this embodiment, typically at least one support wire 84 is fixed to and extends distally from apex clasping catheter 52. The at least one support wire 84 is to be threaded through the at least one suture loop 82, as shown in FIG.15.

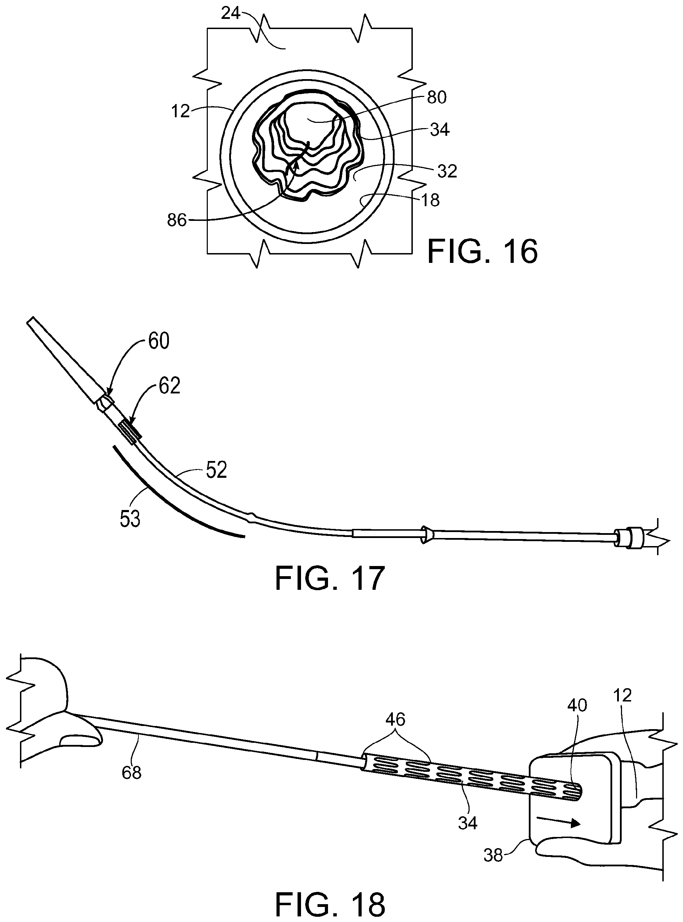

In still another embodiment, taken separately or in combination with any of the other embodiments described herein, radially self-expandable stent graft 34 includes an S-bar support 86 secured to luminal graft component 70 of radially self-expandable stent graft 34, wherein S-bar support 86 is diametrically opposed to longitudinal slot 20 of rigid cylinder 12 when radially self-expandable stent graft 34 is loaded within pocket 32 defined by flexible sheet 24, as can be seen in FIG.16.

In one embodiment, taken separately or in combination with any of the other embodiments described herein, guidewire catheter 54 (shown, for example, in FIG.9) is curved (as can be seen in FIG.17). In one such embodiment, where stent graft includes support-bar 86 (also referred to as an "S-bar" and exemplified, for example in U.S. 8,292,943. As described above, support bar 86 is at a superior position of curvature 53 of apex capture catheter 52, shown in FIG. 17, and to longitudinal slot 20 of rigid cylinder 12.

In another embodiment, the invention is a method of loading a stent graft, such as radially self-expandable stent graft 34, into a stent graft delivery device. In one embodiment of a method of the invention, the method includes loading a radially self-expandable stent graft 34 into pocket 32 defined by a flexible sheet 24 having straight edge 26 and second edge 30 opposite straight edge 26, wherein straight edge 26 has raised component 28 that extends parallel to and is adjacent to longitudinal slot 20 defined by rigid cylinder 12 and extending parallel to longitudinal axis 22 of rigid cylinder 12, flexible sheet 24 extending from raised component 28 of straight edge 26 through longitudinal slot 20 and into lumen 18 defined by rigid cylinder 12 and from within lumen 18 through longitudinal slot 20 of second edge 30 of flexible sheet 24 outside of rigid cylinder 12 to thereby define pocket 32 of flexible sheet 24.

Second edge 30 of flexible sheet 24 is pulled away from rigid cylinder 12, such as by hand, thereby reducing the volume of pocket 32 and radially constricting radially self-expandable stent graft 34, causing longitudinal axis 36 of radially self-expandable stent graft 34 to move away from longitudinal axis 22 of rigid cylinder 12 and toward longitudinal slot 22 of rigid cylinder 12, as described and shown above with reference to FIGs. 5A and 5B and 6A and 6B.

Radially self-expandable stent graft 34 is then directed, along its longitudinal axis 36 from pocket 32 into loading tube 46 while radially self-expandable stent graft 34 is in a radially constricted position. In one embodiment, radially self-expandable stent graft 34 is directed from pocket 32 into loading tube 46 through opening 40 of loading block 38 at one end of rigid cylinder 12, as shown in FIG.18. Opening 40 defined by loading block 38 has a diameter less than that of pocket 32 prior to radially constricting pocket 32 and radially self-expandable stent graft 34, and wherein loading block 38 is beveled at the circumference of opening 40.

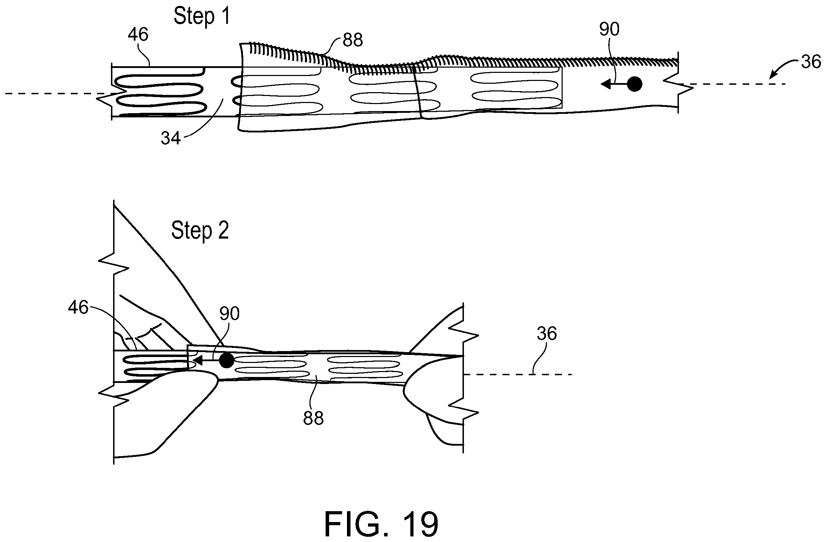

Loading block 38, flexible sheet 24, and rigid cylinder 12 can then be removed, and loading tube 46 is then replaced by flexible sheath 88 of the stent graft delivery device along longitudinal axis 36 of radially self-expandable stent graft 34 while it is radially constricted, as can be seen in the transition from "Step 1" to "Step 2" of FIG.19. More specifically, loading block 38, flexible sheath 24, and rigid cylinder 12 are displaced proximally toward the individual loading the stent graft delivery device over the outside diameter of a primary sheath of the system, proximally as indicated by arrow 90. Stent graft loading system 50, loading block 38, flexible sheath 24, and rigid cylinder 12 are moved away, but not removed from the delivery system until the stent graft is fully loaded into the stent graft delivery system.

In yet another embodiment, the method of the invention includes the step of clasping at least one proximal bare apex 78 of clasping stent 76 of radially self-expandable stent graft 34 to apex clasping component 58 of a stent graft delivery device prior to constricting radially self-expandable stent graft 34 within pocket 32 defined by flexible sheet 24 of stent graft crimping device 10 of the invention, as can be seen in FIG.18.

In one such embodiment, the method further includes the step of removing clasping stent 76 of radially self-expandable stent graft 34 from pocket 32 before clamping proximal bare apex 78 of clasping stent 76 to apex clasping component 58 of the stent graft delivery device, and then redirecting clasping stent 76 back into pocket 32, as can be seen in FIG.9.

In still another embodiment, the method of the invention includes threading at least one support wire 84 of the stent graft delivery device through suture loop 82 of radially self-expandable stent graft 34 prior to radially constricting radially self-expandable stent graft 34, as can be seen in FIGs. 14 and 15. In still another embodiment, the method further includes the step of applying a clamp to threaded suture loop 82 prior to radially constricting radially self-expandable stent graft 34.

Another embodiment of the method, which can be conducted in combination with any of the method steps described above, loading rod 68 is screw-loaded onto distal end 56 of guidewire catheter 54 of a stent graft delivery device, as can be seen in FIG.11.

In yet another embodiment, when radially self-expandable stent graft 34 includes support bar 86, support bar 86 of radially self-expandable stent graft 34 is oriented during loading into pocket 32 radially opposite to longitudinal slot 20 of rigid cylinder 12, as can be seen in FIG.16.

Suitable systems, delivery devices and components of systems, stent grafts as described in U.S. Patent Nos.: 7,763,063; 8,007,605; 8,062,345; 8,062,349; 8,070,790; 8,292,943 and 8,308,790, 8,740,963, 9,198,786, 9,320,631, 9,364,314, and 9,592,112 can be employed to deliver the aortic graft assembly of the invention by the method of the invention.

While example embodiments have been particularly shown and described, will be understood by those skilled in the art in various changes in form and details may be made therein without departing from the scope of the embodiments encompassed by the pendent claims.

## Claims

1. A stent graft crimping device for radially constricting a stent graft, comprising;
a) a rigid cylinder having an outside surface and inside surface, and defining a lumen and a longitudinal slot extending parallel to a longitudinal axis of the rigid cylinder; and
b) a flexible sheet having a straight edge, a raised component at the straight edge, and a second edge opposite the straight edge, the raised component and the straight edge being parallel to and adjacent to the longitudinal slot at the outside surface of the rigid cylinder, the flexible sheet extending from the straight edge and through the slot into the lumen of the rigid cylinder, and from the lumen through the longitudinal slot to the second edge at the outside surface of the rigid cylinder, thereby defining a pocket of the flexible sheet within the lumen of the rigid cylinder.

2. The stent graft crimping device of claim 1, further including a loading block that is mateable to the rigid cylinder and defines an opening in a plane that is perpendicular to the longitudinal axis of the rigid cylinder, the opening defining a center at an axis and parallel to the longitudinal axis of the rigid cylinder, but spaced apart from the longitudinal axis of the rigid cylinder, whereby radial constriction of a stent graft within the pocket defined by the flexible sheet consequent to pulling the second edge of the flexible sheet opposite the straight edge, will cause a longitudinal axis of the stent graft to align with the center of the opening defined by the block, the stent graft thereby being radially constricted and longitudinally moveable from the rigid cylinder through the opening and loaded into a loading tube.

3. A stent graft loading system, comprising:
a) the stent graft crimping device of claim 1;
b) a guidewire catheter extending through the pocket, the catheter having a distal end and a proximal end;
c) an apex clasping component fixed to the distal end of the guidewire catheter, the apex capture component including a distal clasping component at the distal end of the guidewire catheter and a proximal clasping component that is mateable to the distal clasping component and proximal to the distal clasping component; and
d) an apex clasping catheter fixed to and extending proximally from the proximal clasping component of the apex clasping component.

4. The stent graft loading system of claim 3, further including a loading rod that is releasably fixable to the distal end of the guidewire catheter.

5. The stent graft loading system of claim 3, further including a stent graft within the pocket defined by the flexible sheet.

6. The stent graft loading system of claim 5, wherein the stent graft includes a luminal graft component having a proximal end and a distal end, the stent graft including a crown stent fixed to the proximal end of the luminal graft component, and a clasping stent fixed to and within the luminal graft component, the clasping stent being distal to the crown stent and adjacent to the crown stent, the clasping stent including at least one bare apex that is exposed to the lumen of the luminal graft component, and releasably fixable to the apex clasping component.

7. The stent graft loading system of claim 6, wherein the proximal clasping component includes prongs that extend distally from the proximal clasping component and are mateable with the distal clasping component, whereby the at least one apex of the clasping stent can be captured between the prongs of the proximal clasping component and the distal clasping component, and wherein the at least one apex is released by proximal movement of the apex clasping catheter.

8. The stent graft loading system of claim 7, wherein the stent graft includes at least one suture loop at a proximal end of the stent graft and within the stent graft, and wherein at least one support wire is fixed to and extends distally from the apex capture catheter, the at least one support wire being threaded through the at least one suture loop.

9. The stent graft loading system of claim 3, wherein the stent graft includes a support bar, and wherein the support bar of the stent graft is diametrically opposed to the slot of the rigid cylinder.

10. The stent graft loading system of claim 9, wherein the guidewire catheter is curved, and the support bar of the stent graft is at a superior portion of the curvature of the apex capture catheter and to the slot of the rigid cylinder.

11. A method of loading a stent graft into a stent graft delivery device, comprising the steps of:
a) loading a stent graft into a pocket defined by a flexible sheet having a straight edge and a second edge opposite the straight edge, wherein the straight edge has a raised component that extends parallel to and is adjacent a slot defined by a rigid cylinder, and extending parallel to a longitudinal axis of the rigid cylinder, the flexible sheet extending from the raised component of the straight edge through the slot and into a lumen defined by the rigid cylinder, and from within the lumen through the slot to the opposite edge of the flexible sheet outside of the rigid cylinder to thereby define the pocket of the flexible sheet;
b) pulling the second edge of the flexible sheet away from the rigid cylinder, thereby reducing the volume of the pocket and radially constricting the stent graft and causing a longitudinal axis of the stent graft to move away from the longitudinal axis of the rigid cylinder and toward the slot of the rigid cylinder; and
c) directing the stent graft, while it is radially constricted thereby forming a radially constricted stent graft, along its longitudinal axis from the pocket through an opening defined by a block and into a loading tube, while remaining radially constricted along its longitudinal axis.

12. The method of claim 11, wherein the step of directing the stent graft, while it is radially constricted, from the pocket includes directing the radially constricted stent graft through an opening at the block at one end of the rigid cylinder.

13. The method of claim 12, wherein the opening defined by the block has a diameter less than that of the pocket prior to radially constricting the pocket and the stent graft, and wherein the block is beveled at a circumference of the opening.

14. The method of claim 12, wherein the loading tube is intermediate to a stent graft delivery device, whereby the radially constricted stent graft is directed from the radially-constricted member to the stent graft delivery device along the longitudinal axis of the radially constricted stent graft while radially constricted.

15. The method of claim 14, further including the step of clasping at least one proximal apex of a clasping stent of the stent graft to an apex-clasping component of a stent graft delivery device prior to constricting the stent graft within the pocket of the flexible sheet.

16. The method of claim 15, further including the step of releasing the clasping stent of the radially constricted stent graft from the pocket before clamping the proximal apex of the clasping stent to the apex-clasping component of the stent graft delivery device, and then redirecting the radially constricted stent graft back into the pocket;
further including the step of threading at least one support wire of the stent graft delivery device through a suture of the stent graft prior to radially constricting the stent graft;
further including the step of applying a clamp to the threaded suture prior to radially constricting the stent graft;
further including the step of screw-loading a rod onto threads at a distal end of the guidewire catheter;
wherein the stent graft includes a support bar, and wherein the support bar of the stent graft is oriented during loading into the pocket radially opposite to the longitudinal slot of the rigid cylinder.

## Patentansprüche

1. Stentimplantat-Komprimiervorrichtung zum radialen Verengen eines Stentimplantats, umfassend;
a) einen starren Zylinder mit einer Außenfläche und einer Innenfläche, der ein Lumen und einen Längsschlitz definiert, der sich parallel zu einer Längsachse des starren Zylinders erstreckt; und
b) eine flexible Bahn mit einer geraden Kante, einer erhöhten Komponente an der geraden Kante und einer zweiten Kante gegenüber der geraden Kante, wobei die erhöhte Komponente und die gerade Kante zu dem Längsschlitz an der Außenfläche des starren Zylinders parallel und benachbart sind, wobei sich die flexible Bahn von der geraden Kante und durch den Schlitz in das Lumen des starren Zylinders und aus dem Lumen durch den Längsschlitz zu der zweiten Kante an der Außenfläche des starren Zylinders erstreckt und auf diese Weise eine Tasche der flexiblen Bahn in dem Lumen des starren Zylinders definiert.

2. Stentimplantat-Komprimiervorrichtung nach Anspruch 1, ferner beinhaltend einen Ladeblock, der mit dem starren Zylinder zusammensteckbar ist und eine Öffnung in einer Ebene senkrecht zu der Längsachse des starren Zylinders definiert, wobei die Öffnung eine Mitte an einer Achse und parallel zu der Längsachse des starren Zylinders, aber beabstandet von der Längsachse des starren Zylinders definiert, wobei eine radiale Verengung eines Stentimplantats in der durch die flexible Bahn definierten Tasche als Folge des Ziehens der zweiten Kante der flexiblen Bahn gegenüber der geraden Kante bewirkt, dass sich eine Längsachse des Stentimplantats an der Mitte der durch den Block definierten Öffnung ausrichtet, wodurch das Stentimplantat radial verengt wird und in Längsrichtung aus dem starren Zylinder durch die Öffnung bewegbar ist und in eine Laderöhre geladen wird.

3. Stentimplantat-Ladesystem, umfassend:
a) die Stentimplantat-Komprimiervorrichtung nach Anspruch 1;
b) einen Führungsdrahtkatheter, der sich durch die Tasche erstreckt, wobei der Katheter ein distales Ende und ein proximales Ende aufweist;
c) eine Scheitelpunkteinklemmkomponente, die an dem distalen Ende des Führungsdrahtkatheters fixiert ist, wobei die Scheitelpunkteinfangkomponente eine distale Einklemmkomponente am distalen Ende des Führungsdrahtkatheters und eine proximale Einklemmkomponente beinhaltet, die mit der distalen Einklemmkomponente zusammensteckbar und proximal zu der distalen Einklemmkomponente ist; und
d) einen Scheitelpunkteinklemmkatheter, der an der proximalen Einklemmkomponente der Scheitelpunkteinklemmkomponente fixiert ist und sich proximal von dieser erstreckt.

4. Stentimplantat-Ladesystem nach Anspruch 3, ferner beinhaltend einen Ladestange, die lösbar an dem distalen Ende des Führungsdrahtkatheters fixierbar ist.

5. Stentimplantat-Ladesystem nach Anspruch 3, ferner beinhaltend ein Stentimplantat in der durch die flexible Bahn definierten Tasche.

6. Stentimplantat-Ladesystem nach Anspruch 5, wobei das Stentimplantat eine luminale Implantatkomponente mit einem proximalen Ende und einem distalen Ende beinhaltet, wobei das Stentimplantat einen Kronen-Stent, der an dem proximalen Ende der luminalen Implantatkomponente fixiert ist, und einen Einklemm-Stent, der an der luminalen Implantatkomponente fixiert ist und sich innerhalb dieser befindet, beinhaltet, wobei der Einklemm-Stent zu dem Kronen-Stent distal und zu dem Kronen-Stent benachbart ist, wobei der Einklemm-Stent wenigstens einen bloßliegenden Scheitelpunkt beinhaltet, der zum Lumen der luminalen Implantatkomponente freiliegt, und lösbar an der Scheitelpunkteinklemmkomponente fixierbar ist.

7. Stentimplantat-Ladesystem nach Anspruch 6, wobei die proximale Einklemmkomponente Zinken beinhaltet, die sich distal von der proximalen Einklemmkomponente erstrecken und mit der distalen Einklemmkomponente zusammensteckbar sind, wodurch der wenigstens eine Scheitelpunkt des Einklemm-Stents zwischen den Zinken der proximalen Einklemmkomponente und der distalen Einklemmkomponente eingefangen werden kann, und wobei der wenigstens eine Scheitelpunkt durch eine proximale Bewegung des Scheitelpunkteinklemmkatheters gelöst wird.

8. Stentimplantat-Ladesystem nach Anspruch 7, wobei das Stentimplantat wenigstens eine Nahtschlaufe an einem proximalen Ende des Stentimplantats und in dem Stentimplantat beinhaltet und wobei wenigstens ein Trägerdraht an dem Scheitelpunkteinfangkatheter fixiert ist und sich distal von diesem erstreckt, wobei der wenigstens eine Trägerdraht durch die wenigstens eine Nahtschlaufe eingefädelt ist.

9. Stentimplantat-Ladesystem nach Anspruch 3, wobei das Stentimplantat eine Trägerleiste umfasst und wobei die Trägerleiste des Stentimplantats dem Schlitz des starren Zylinders diametral gegenüberliegt.

10. Stentimplantat-Ladesystem nach Anspruch 9, wobei der Führungsdrahtkatheter gekrümmt ist und sich die Trägerleiste des Stentimplantats an einem oberen Abschnitt der Krümmung des Scheitelpunkteinfangkatheters und zu dem Schlitz des starren Zylinders befindet.

11. Verfahren zum Laden eines Stentimplantats in eine Stentimplantat-Abgabevorrichtung, folgende Schritte umfassend:
a) Laden eines Stentimplantats in eine Tasche, die durch eine flexible Bahn definiert wird, die eine gerade Kante und eine zweite Kante gegenüber der geraden Kante aufweist, wobei die gerade Kante eine erhöhte Komponente aufweist, die sich parallel zu einem durch einen starren Zylinder definierten Schlitz erstreckt und zu diesem benachbart ist, und sich parallel zu einer Längsachse des starren Zylinders erstreckt, wobei sich die flexible Bahn von der erhöhten Komponente der geraden Kante durch den Schlitz und in ein durch den starren Zylinder definiertes Lumen und aus dem Lumen durch den Schlitz zur gegenüberliegenden Kante der flexiblen Bahn aus dem starren Zylinder heraus erstreckt, um auf diese Weise die Tasche der flexiblen Bahn zu definieren;
b) Wegziehen der zweiten Kante der flexiblen Bahn von dem starren Zylinder, wodurch das Volumen der Tasche reduziert wird und das Stentimplantat radial verengt wird und bewirkt wird, dass sich eine Längsachse des Stentimplantats von der Längsachse des starren Zylinders weg zu dem Schlitz des starren Zylinders hin bewegt; und
c) Lenken des Stentimplantats, während es radial verengt ist, wodurch ein radial verengtes Stentimplantat gebildet wird, entlang seiner Längsachse aus der Tasche durch eine Öffnung, die durch einen Block definiert ist, und in eine Laderöhre, während es entlang seiner Längsachse radial verengt bleibt.

12. Verfahren nach Anspruch 11, wobei der Schritt des Lenkens des Stentimplantats, während es radial verengt ist, aus der Tasche das Lenken des radial verengten Stentimplantats durch eine Öffnung an dem Block an einem Ende des starren Zylinders beinhaltet.

13. Verfahren nach Anspruch 12, wobei die durch den Block definierte Öffnung einen kleineren Durchmesser als die Tasche vor dem radialen Verengen der Tasche und des Stentimplantats aufweist und wobei der Block an einem Umfang der Öffnung abgeschrägt ist.

14. Verfahren nach Anspruch 12, wobei die Laderöhre zu einer Stentimplantat-Abgabevorrichtung intermediär ist, wodurch das radial verengte Stentimplantat unter radialer Verengung entlang der Längsachse des radial verengten Stentimplantats aus dem radial verengten Element zu der Stentimplantat-Abgabevorrichtung gelenkt wird.

15. Verfahren nach Anspruch 14, ferner beinhaltend den Schritt des Einklemmens von wenigstens einem proximalen Scheitelpunkt eines Einklemm-Stents des Stentimplantats an einer Scheitelpunkteinklemmkomponente einer Stentimplantat-Abgabevorrichtung vor dem Verengen des Stentimplantats in der Tasche der flexiblen Bahn.

16. Verfahren nach Anspruch 15, ferner beinhaltend den Schritt des Lösens des Einklemm-Stents des radial verengten Stentimplantats aus der Tasche vor dem Einklemmen des proximalen Scheitelpunkts des Einklemm-Stents an der Scheitelpunkteinklemmkomponente der Stentimplantat-Abgabekomponente und des anschließenden Umlenkens des radial verengten Stentimplantats zurück in die Tasche;
ferner beinhaltend den Schritt des Einfädelns wenigstens eines Trägerdrahts der Stentimplantat-Abgabevorrichtung durch eine Naht des Stentimplantats vor dem radialen Verengen des Stentimplantats;
ferner beinhaltend den Schritt des Aufbringens einer Klammer auf die eingefädelte Naht vor dem radialen Verengen des Stentimplantats;
ferner beinhaltend den Schritt des Aufschraubens einer Stange auf ein Gewinde an einem distalen Ende des Führungsdrahtkatheters;
wobei das Stentimplantat eine Trägerleiste beinhaltet und wobei die Trägerleiste des Stentimplantats während des Ladens in die Tasche radial gegenüber dem Längsschlitz des starren Zylinders ausgerichtet wird.

## Revendications

1. Dispositif de sertissage de greffe d'endoprothèse pour contraindre radialement une greffe d'endoprothèse, comprenant ;
a) un cylindre rigide ayant une surface extérieure et une surface intérieure, et définissant une lumière et une fente longitudinale se prolongeant parallèlement à un axe longitudinal du cylindre rigide ; et
b) une feuille flexible ayant un bord droit, un composant surélevé au niveau du bord droit, et un second bord opposé au bord droit, le composant surélevé et le bord droit étant parallèles et adjacents à la fente longitudinale au niveau de la surface extérieure du cylindre rigide, la feuille flexible se prolongeant à partir du bord droit et à travers la fente dans la lumière du cylindre rigide, et à partir de la lumière à travers la fente longitudinale jusqu'au second bord au niveau de la surface extérieure du cylindre rigide, définissant ainsi une poche de la feuille flexible à l'intérieur de la lumière du cylindre rigide.

2. Dispositif de sertissage de greffe d'endoprothèse selon la revendication 1, comportant également un bloc de chargement qui peut s'accoupler au cylindre rigide et définit une ouverture dans un plan qui est perpendiculaire à l'axe longitudinal du cylindre rigide, l'ouverture définissant un centre au niveau d'un axe et parallèle à l'axe longitudinal du cylindre rigide, mais espacé de l'axe longitudinal du cylindre rigide, moyennant quoi la contrainte radiale d'une greffe d'endoprothèse à l'intérieur de la poche définie par la feuille flexible consécutive à la traction du second bord de la feuille flexible opposé au bord droit amènera un axe longitudinal de la greffe d'endoprothèse à s'aligner avec le centre de l'ouverture définie par le bloc, la greffe d'endoprothèse étant ainsi radialement contrainte et longitudinalement mobile à partir du cylindre rigide à travers l'ouverture et chargée dans un tube de chargement.

3. Système de chargement de greffe d'endoprothèse, comprenant :
a) le dispositif de sertissage de greffe d'endoprothèse selon la revendication 1 ;
b) un cathéter à fil-guide se prolongeant à travers la poche, le cathéter ayant une extrémité distale et une extrémité proximale ;
c) un composant de préhension de l'apex fixé à l'extrémité distale du cathéter à fil-guide, le composant de capture de l'apex comportant un composant de préhension distal au niveau de l'extrémité distale du cathéter à fil-guide et un composant de préhension proximal qui peut s'accoupler au composant de préhension distal et proximal au composant de préhension distal ; et
d) un cathéter de préhension de l'apex fixé au et se prolongeant de manière proximale à partir du composant de préhension proximal du composant de préhension de l'apex.

4. Système de chargement de greffe d'endoprothèse selon la revendication 3, comportant également une tige de chargement qui peut se fixer de manière amovible à l'extrémité distale du cathéter à fil-guide.

5. Système de chargement de greffe d'endoprothèse selon la revendication 3, comportant également une greffe d'endoprothèse à l'intérieur de la poche définie par la feuille flexible.

6. Système de chargement de greffe d'endoprothèse selon la revendication 5, dans lequel la greffe d'endoprothèse comporte un composant de greffe luminal ayant une extrémité proximale et une extrémité distale, la greffe d'endoprothèse comportant un stent en couronne fixé à l'extrémité proximale du composant de greffe luminal, et un stent de préhension fixé à et à l'intérieur du composant de greffe luminal, l'endoprothèse de préhension étant distale par rapport au stent en couronne et adjacente au stent en couronne, l'endoprothèse de préhension comportant au moins un apex nu qui est exposé à la lumière du composant de greffe luminal, et pouvant se fixer de manière amovible au composant de préhension de l'apex.

7. Système de chargement de greffe d'endoprothèse selon la revendication 6, dans lequel le composant de préhension proximal comporte des branches qui se prolongent distalement à partir du composant de préhension proximal et peuvent s'accoupler au composant de préhension distal, moyennant quoi l'au moins un apex de l'endoprothèse de préhension peut être capturé entre les branches du composant de préhension proximal et le composant de préhension distal, et dans lequel l'au moins un apex est libéré par le mouvement proximal du cathéter de préhension de l'apex.

8. Système de chargement de greffe d'endoprothèse selon la revendication 7, dans lequel la greffe d'endoprothèse comporte au moins une boucle de suture au niveau d'une extrémité proximale de la greffe d'endoprothèse et à l'intérieur de la greffe d'endoprothèse, et dans lequel au moins un fil de support est fixé au cathéter de capture de l'apex et se prolonge distalement à partir de celui-ci, l'au moins un fil de support étant fileté à travers l'au moins une boucle de suture.

9. Système de chargement de greffe d'endoprothèse selon la revendication 3, dans lequel la greffe d'endoprothèse comporte une barre de support, et dans lequel la barre de support de la greffe d'endoprothèse est diamétralement opposée à la fente du cylindre rigide.

10. Système de chargement de greffe d'endoprothèse selon la revendication 9, dans lequel le cathéter à fil-guide est courbé, et la barre de support de la greffe d'endoprothèse est au niveau d'une partie supérieure de la courbure du cathéter de capture de l'apex et à la fente du cylindre rigide.

11. Procédé de chargement d'une greffe d'endoprothèse dans un dispositif de mise en place de greffe d'endoprothèse, comprenant les étapes suivantes :
a) le chargement d'une greffe d'endoprothèse dans une poche définie par une feuille flexible ayant un bord droit et un second bord opposé au bord droit, dans lequel le bord droit a un composant surélevé qui se prolonge parallèlement à et est adjacent à une fente définie par un cylindre rigide, et se prolongeant parallèlement à un axe longitudinal du cylindre rigide, la feuille flexible se prolongeant à partir du composant surélevé du bord droit à travers la fente et dans une lumière définie par le cylindre rigide, et à partir de l'intérieur de la lumière à travers la fente jusqu'au bord opposé de la feuille flexible à l'extérieur du cylindre rigide pour ainsi définir la poche de la feuille flexible ;
b) la traction du second bord de la feuille flexible à l'écart du cylindre rigide, réduisant ainsi le volume de la poche et contraignant radialement la greffe d'endoprothèse et amenant un axe longitudinal de la greffe d'endoprothèse à s'éloigner de l'axe longitudinal du cylindre rigide et vers la fente du cylindre rigide ; et
c) la direction de la greffe d'endoprothèse, tandis qu'elle est radialement contrainte formant ainsi une greffe d'endoprothèse radialement contrainte, le long de son axe longitudinal à partir de la poche à travers une ouverture définie par un bloc et dans un tube de chargement, tout en restant radialement contrainte le long de son axe longitudinal.

12. Procédé selon la revendication 11, dans lequel l'étape de direction de la greffe d'endoprothèse, tandis qu'elle est radialement contrainte, à partir de la poche comporte la direction de la greffe d'endoprothèse radialement contrainte à travers une ouverture au niveau du bloc au niveau d'une extrémité du cylindre rigide.

13. Procédé selon la revendication 12, dans lequel l'ouverture définie par le bloc a un diamètre inférieur à celui de la poche avant de contraindre radialement la poche et la greffe d'endoprothèse, et dans lequel le bloc est biseauté au niveau d'une circonférence de l'ouverture.

14. Procédé selon la revendication 12, dans lequel le tube de chargement est intermédiaire à un dispositif de mise en place de greffe d'endoprothèse, moyennant quoi la greffe d'endoprothèse radialement contrainte est dirigée à partir de l'élément radialement contraint jusqu'au dispositif de mise en place de greffe d'endoprothèse le long de l'axe longitudinal de la greffe d'endoprothèse radialement contrainte tout en étant radialement contrainte.

15. Procédé selon la revendication 14, comportant également l'étape de préhension d'au moins un apex proximal d'une endoprothèse de préhension de la greffe d'endoprothèse à un composant de préhension de l'apex d'un dispositif de mise en place de greffe d'endoprothèse avant de contraindre la greffe d'endoprothèse à l'intérieur de la poche de la feuille flexible.

16. Procédé selon la revendication 15, comportant également l'étape de libération de l'endoprothèse de préhension de la greffe d'endoprothèse radialement contrainte de la poche avant de serrer l'apex proximal de l'endoprothèse de préhension au composant de préhension de l'apex du dispositif de mise en place de greffe d'endoprothèse, puis de redirection de la greffe d'endoprothèse radialement contrainte de nouveau dans la poche ;
comportant également l'étape de filetage d'au moins un fil de support du dispositif de mise en place de greffe d'endoprothèse à travers une suture de la greffe d'endoprothèse avant de contraindre radialement la greffe d'endoprothèse ;
comportant également l'étape d'application d'une pince sur la suture filetée avant de contraindre radialement la greffe d'endoprothèse ;
comportant également l'étape de vissage d'une tige sur des filetages au niveau d'une extrémité distale du cathéter à fil-guide ;
dans lequel la greffe d'endoprothèse comporte une barre de support, et dans lequel la barre de support de la greffe d'endoprothèse est orientée pendant le chargement dans la poche radialement opposée à la fente longitudinale du cylindre rigide.
